# EUROPEAN PATENT APPLICATION

(11) **EP 4 691 355 A1**
(43) Date of publication of application: **11.02.2026**
(21) Application number: 24807048.4
(22) Date of filing: 30.04.2024
(51) Int. Cl.: A61B 5/022

(54) **SPHYGMOMANOMETER, BLOOD PRESSURE MEASUREMENT METHOD, BLOOD PRESSURE MEASUREMENT PROGRAM, LEARNING MODEL CONSTRUCTION METHOD, AND LEARNING MODEL CONSTRUCTION PROGRAM**

(30) Priority: 12.05.2023 JP 2023079663
(71) Applicant: OMRON Corporation, Kyoto-shi, Kyoto 600-8530 (JP)
(72) Inventor: YANAGAWA, Yukiko, Kyoto-shi, Kyoto 600-8530 (JP); NISHIYUKI, Kenta, Kyoto-shi, Kyoto 600-8530 (JP); WADA, Hirotaka, Kyoto-shi, Kyoto 600-8530 (JP); UCHIDA, Shihori, Kyoto-shi, Kyoto 600-8530 (JP); KOIZUMI, Masayuki, Kyoto-shi, Kyoto 600-8530 (JP)
(74) Representative: Isarpatent
(86) International application number: PCT/JP2024/016716
(87) International publication number: WO 2024/237094

(57) **Abstract**

The sphygmomanometer includes: a cuff; a control unit that controls pressure applied to the arm by the cuff; a detection unit that detects cuff pressure; a measurement unit that measures blood pressure of the measurement subject by an oscillometric method by using a time-series change in the cuff pressure; a correction model, for multiple subjects, constructed by machine learning using information of a waveform related to the time-series change in the cuff pressure as an explanatory variable and using an error of blood pressure measured by the measurement unit with respect to blood pressure adopted as a true value as an objective variable; a correction unit that corrects the blood pressure based on an error output by the correction model by inputting the information of the waveform to the correction model; and an output unit that outputs the blood pressure corrected by the correction unit.

## Description

### TECHNICAL FIELD

The present invention relates to a sphygmomanometer, a blood pressure measurement method, a blood pressure measurement program, a learning model construction method, and a learning model construction program.

### BACKGROUND OF INVENTION

Health condition is checked by blood pressure measurement. Patent Document 1 describes a technique of storing a correlation between blood pressure of a measurement subject measured by using a cuff and blood pressure of the measurement subject actually measured by an auscultation method, and correcting the blood pressure of the measurement subject by using the stored correlation. Further, Patent Document 2 describes a technique for measuring blood pressure by using a photoplethysmogram.

### CITATION LIST

### PATENT LITERATURE

Patent Document 1: JP 5-309073 A
Patent Document 2: JP 2022-516820 T

### SUMMARY

### TECHNICAL PROBLEM

In blood pressure measurement by the oscillometric method, a cuff wound around the upper arm or wrist of a measurement subject is pressurized, and then pressure oscillation superimposed on the internal pressure of the cuff is detected while gradually reducing the pressure, and the blood pressure is measured based on the detected pressure oscillation. Here, noise may be intermingled with the detected pressure oscillation depending on a body motion of the measurement subject, a way of winding the cuff, a position where the cuff is wound, and the like. Such noise interminglement may cause a decrease in accuracy of blood pressure measurement by the oscillometric method. Here, it is conceivable to suppress the decrease in accuracy of blood pressure measurement by machine learning, but it is not easy to collect a large number of pieces of training data that can suppress the influence of noise.

An object of one aspect of the disclosed technology is to provide a sphygmomanometer, a blood pressure measurement method, a blood pressure measurement program, a learning model construction method, and a learning model construction program that are capable of achieving highly accurate blood pressure measurement even with a small amount of training data.

### SOLUTION TO PROBLEM

One aspect of the disclosed technique is exemplified by the following sphygmomanometer. The present sphygmomanometer includes: a cuff to be wound around an arm of a measurement subject; a control unit configured to control pressure applied to the arm by the cuff; a detection unit configured to detect cuff pressure received by the cuff from the arm; a measurement unit configured to measure blood pressure of the measurement subject by an oscillometric method by using a time-series change in the cuff pressure; a correction model, for a subject, constructed by machine learning using information of a waveform related to the time-series change in the cuff pressure as an explanatory variable and using an error of blood pressure measured by the measurement unit with respect to blood pressure adopted as a true value as an objective variable; a correction unit configured to correct the blood pressure measured by the measurement unit based on an error output by the correction model by inputting the information of the waveform related to the time-series change in the cuff pressure detected by the detection unit to the correction model; and an output unit configured to output the blood pressure corrected by the correction unit.

According to the sphygmomanometer above, since the blood pressure measured by the oscillometric method is corrected by using the error above, the measurement accuracy of the blood pressure can be improved. Further, in the sphygmomanometer, the error of the blood pressure measured by the oscillometric method with respect to the blood pressure adopted as the true value is used as the objective variable. The range of the error is considered to be approximately ±10 mmHg, and the range of the maximum blood pressure of a person is considered to be approximately from 80 mmHg to 200 mmHg. In machine learning, the number of pieces of training data tends to increase in proportion to the size of the variable range of an objective variable. In other words, the number of pieces of training data used for learning can be reduced by making the variable range of an objective variable smaller. The sphygmomanometer uses the error having a narrower variable range than the maximum blood pressure as the objective variable, and thus can reduce the number of pieces of training data used for learning. That is, the present sphygmomanometer can enable highly accurate blood pressure measurement even with a small amount of training data. Note that the blood pressure adopted as the true value may be blood pressure actually measured by the auscultation method.

Here, the information of the waveform may be, for example, information related to the cuff pressure. The measurement unit may acquire an OscilloMetric Waveform (OMW) based on a time-series change in the cuff pressure, and the information of the waveform may include OMW information indicating a waveform of the OMW.

The measurement unit may acquire an OscilloMetric Waveform Envelope (OMWE) based on the OMW, and the information of the waveform may include OMWE information indicating a waveform of the OMWE.

Here, the present sphygmomanometer may correct the blood pressure measured by the measurement unit based on an error output by the correction model by inputting both the time-series change in the cuff pressure and the OMW information to the correction model. The present sphygmomanometer may correct the blood pressure measured by the measurement unit based on an error output by the correction model by inputting the time-series change in the cuff pressure and the OMWE information to the correction model.

The disclosed technology can also be understood from aspects of a blood pressure measurement method, a blood pressure measurement program, a learning model construction method, and a learning model construction program.

### ADVANTAGEOUS EFFECTS OF INVENTION

According to the disclosed technique, it is possible to achieve highly accurate blood pressure measurement even with a small amount of training data.

### BRIEF DESCRIPTION OF THE DRAWINGS

FIG. 1 is a diagram illustrating an example of a sphygmomanometer according to the present embodiment.
FIG. 2 is a diagram schematically illustrating correction of blood pressure by a correction unit according to the embodiment.
FIG. 3 is a diagram schematically illustrating construction of a correction model according to the embodiment.
FIG. 4 is a diagram illustrating an example of a processing flow of the sphygmomanometer according to the embodiment.
FIG. 5 is a diagram exemplifying a verification result of measurement accuracy of blood pressure.
FIG. 6 is a diagram illustrating an example of a sphygmomanometer according to a first modified example.

### DESCRIPTION OF EMBODIMENTS

### <Application Example>

An application example of the present invention will be described. A sphygmomanometer 100 according to the application example is a device that measures the blood pressure of a measurement subject by winding a cuff 120 around the arm of the measurement subject. The cuff 120 is wound around, for example, an upper arm, a wrist, or the like of the arm of the measurement subject. That is, the sphygmomanometer 100 may be a wrist type sphygmomanometer. In the sphygmomanometer 100, a measurement unit 114 measures the blood pressure of the measurement subject by an oscillometric method. The measurement unit 114 inputs the measured blood pressure of the measurement subject to a correction unit 115.

The correction unit 115 corrects the blood pressure measured by the measurement unit 114 by using a correction model 116. The correction model 116 is a learning model constructed by machine learning in which information of a waveform related to a time-series change in the cuff pressure by the cuff 120 is used as an explanatory variable and an error of the blood pressure measured by the measurement unit 114 with respect to the blood pressure adopted as a true value is used as an objective variable. Information of a waveform related to the time-series change in the cuff pressure includes information of a waveform of OscilloMetric Waveform (OMW) and information of a waveform from OscilloMetric Waveform Envelope (OMWE). The measurement unit 114 measures blood pressure by, for example, an oscillometric method. Note that the blood pressure adopted as the true value may be, for example, blood pressure actually measured by an auscultation method. Machine learning also includes deep learning.

When the information of the waveform related to the time-series change of the cuff pressure is input, the correction model 116 outputs an error of the blood pressure measured by the oscillometric method with respect to the blood pressure adopted as the true value. The correction unit 115 corrects the blood pressure measured by the measurement unit 114 by using the error output from the correction model 116. In the sphygmomanometer 100, the blood pressure measured by the measurement unit 114 can be made as close as possible to the blood pressure actually measured by the auscultation method with the correction described above.

Here, as described above, the correction model 116 in the present application example uses, as an objective variable, an error of the blood pressure measured by the oscillometric method with respect to the blood pressure adopted as the true value. The error is considered to be within a range of approximately ±10 mmHg. On the other hand, the value of the maximum blood pressure of a person is in the range of approximately 80 mmHg to 200 mmHg.

That is, when the blood pressure is used as the objective variable of the correction model 116, the variable range of the objective variable is about 10 times as large as that when the error is used as the objective variable. In the machine learning, the number of pieces of training data tends to increase in proportion to the size of the variable range of the objective variable. Hence, in the present application example in which the error is used as the objective variable, it is possible to reduce the number of pieces of training data used for constructing the correction model 116 compared with a case in which the blood pressure is used as the objective variable. That is, according to the present application example, it is possible to achieve highly accurate blood pressure measurement even with a small amount of training data.

### <Embodiments>

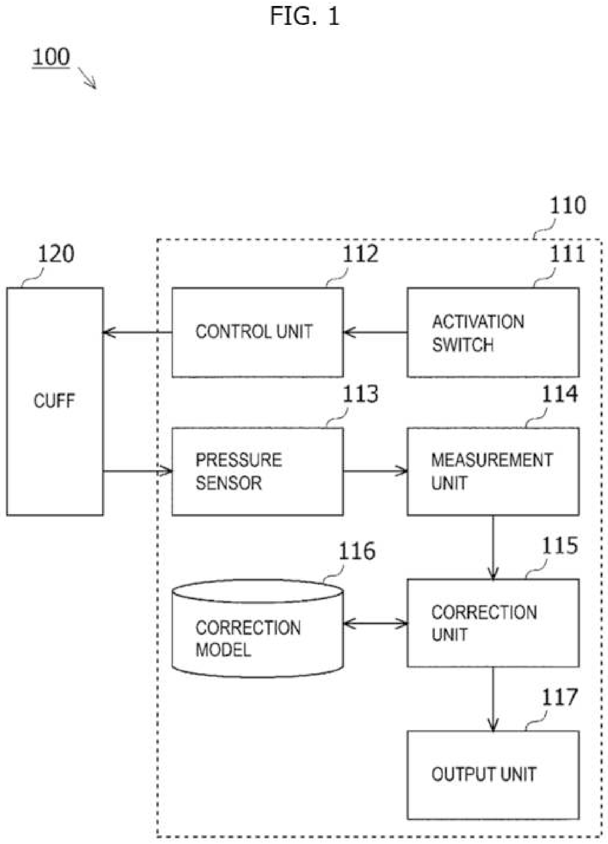

Next, embodiments will be described below. FIG. 1 is a diagram illustrating an example of the sphygmomanometer 100 according to the present embodiment. The sphygmomanometer 100 includes a main body 110 and the cuff 120. The sphygmomanometer 100 measures the blood pressure of the measurement subject by winding the cuff 120 around the upper arm or the wrist (hereinafter also referred to as the arm) of the measurement subject.

The cuff 120 is formed in a belt shape and has a bag into which air is inhaled. When air is inhaled into the bag inside the cuff 120 in a state that the cuff 120 is wound around the arm of the measurement subject, the pressure by the cuff on the arm increases. When air is exhaled from the bag inside the cuff 120 in the state that the cuff 120 is wound around the arm of the measurement subject, the pressure by the cuff on the arm decreases.

A processor and a storage unit, for example, are disposed in the main body 110, and the processor executes a program stored in the storage unit, thereby implementing each processing unit such as an activation switch 111, a control unit 112, a pressure sensor 113, the measurement unit 114, the correction unit 115, the correction model 116, and an output unit 117. In other words, the sphygmomanometer 100 can be regarded as a computer having the cuff 120. Note that each of the processing units such as the activation switch 111, the control unit 112, the pressure sensor 113, the measurement unit 114, the correction unit 115, the correction model 116, and the output unit 117 may be a dedicated hardware circuit. In the sphygmomanometer 100, the sphygmomanometer 100 is activated by turning on the activation switch 111, and measurement of blood pressure is started.

The control unit 112 controls the pressure applied to the arm of the measurement subject by the cuff 120 by inhaling air into the cuff 120 and exhaling air from the cuff 120. When the activation switch 111 is turned on, the control unit 112 starts controlling the pressure of the cuff 120.

The pressure sensor 113 is a sensor that detects the pressure (cuff pressure) by the cuff 120. The pressure sensor 113 outputs the detected cuff pressure to the measurement unit 114. The cuff pressure includes, for example, the pressure applied to the arm by the cuff 120 and pressure oscillation because of pulsation of blood flow flowing in the artery of the arm.

The measurement unit 114 performs blood pressure measurement by an oscillometric method based on the pressure detected by the pressure sensor 113. When the pressure by the cuff 120 becomes higher than the maximum blood pressure, the artery is occluded, and the pressure oscillation because of the pulsation of the blood flow does not occur. On the other hand, when the pressure by the cuff 120 becomes lower than the maximum blood pressure, the artery starts to open, and pressure oscillation because of pulsation of the blood flow is superimposed on the pressure by the cuff 120. When the pressure by the cuff 120 further decreases to be less than the minimum blood pressure, the artery inside the arm deforms so as to slightly swell from the open state, and the pressure oscillation does not occur.

The measurement unit 114 acquires, from the pressure sensor 113, a time-series change in the cuff pressure of the cuff 120 that is increased to pressure higher than the maximum blood pressure and then gradually decreased to pressure lower than the minimum blood pressure. The measurement unit 114 extracts, from the time-series change in cuff pressure acquired from the pressure sensor 113, a time-series change in pressure oscillation because of pulsation of the blood flow flowing through the artery of the blood flow. The extracted time-series change of the pressure oscillation is the OMW. The measurement unit 114 further extracts the OMWE from the OMW. The measurement unit 114 measures the blood pressure of the measurement subject by using the feature amount extracted from the OMW (hereinafter also referred to as an OMW feature amount) and the feature amount extracted from the OMWE (hereinafter also referred to as an OMWE feature amount). The measurement unit 114 may measure the blood pressure by the oscillometric method by using either one of the OMW feature amount and the OMWE feature amount. Further, the measurement unit 114 outputs information of the waveform related to the time-series change in the cuff pressure to the correction unit 115. The information of the waveform related to the time-series change in the cuff pressure includes OMW information indicating the waveform of the OMW and OMWE information indicating the waveform of the OMWE. In the following, there will be described a case, as an example, in which at least one of the OMW information and the OMWE information is used as the information of the waveform related to the time-series change in the cuff pressure.

As the OMW information and the OMWE information output from the measurement unit 114 include the following information from (1) to (28), for example. The information from (1) to (14) can be information related to the waveform shape of the OMW or the OMWE. Further, each piece of information from (15) to (20) can be information related to the cuff pressure.
(1) Minimum value, mean value, minimum value.
(2) The value at a time point when a time axis is X% (X is a numerical value from 0 to 100).
(3) The time at which the value is the maximum.
(4) The area of a region surrounded by the waveform of the OMW or the OMWE and the time axis.
(5) The kurtosis and skewness of the waveform of the OMW or the OMWE.
(6) The number of peaks in the waveform of the OMW or the OMWE.
(7) The complexity of the waveform of the OMW or the OMWE (for example, the sum of squares of differences between before and after in the time-series data).
(8) The number of points exceeding the mean value of the waveform of the OMW or the OMWE up to a predetermined time point (for example, time point of 1/3 from the start).
(9) The waveform of the OMW or the OMWE is divided into a predetermined number (for example, "4") of regions, and the gradient in each of the divided regions.
(10) A value obtained by dividing the time length before the peak by the time length after the peak of the waveform of the OMW or the OMWE.
(11) The change rate in the height of the waveform of the OMW or the OMWE.
(12) The waveform of the OMW or the OMWE is divided into a predetermined number (for example, "4") of regions, and the area ratio of the waveform to the circumscribed rectangle for each of the divided regions.
(13) A value obtained by subtracting the peak time of the lower envelope of the OMW from the peak time of the upper envelope of the OMW.
(14) The area ratio of the area of the upper envelope of the OMW and the area of the lower envelope of the OMW.
(15) The cuff pressure at the OMWE peak.
(16) The cuff pressure at a point where the first derivative of the OMWE is the maximum and the cuff pressure at a point where the first derivative of the OMWE is the minimum.
(17) In the first half of the peak of the OMWE, the cuff pressure at a point N times (N is an integer greater than 0 and less than 1) lower than the peak.
(18) In the second half of the peak of the OMWE, the cuff pressure at a point N times (N is an integer greater than 0 and less than 1) lower than the peak.
(19) In the OMWE, time from the point at which the first derivative is the maximum to the peak.
(20) In the OMWE, time from the peak to the point at which the first derivative is the minimum.
(21) The time length of the OMWE.
(22) A value that the time to the OMWE peak divided by the length of time of the OMWE.
(23) The position of the mean "-σ" when the OMWE is Gaussian-fitted.
(24) The position of the mean "+σ" when the OMWE is Gaussian-fitted.
(25) The value of the OMWE at the position of the mean "-σ" when the OMWE is Gaussian-fitted.
(26) The value of the OMWE at the position of the mean "+σ" when the OMWE is Gaussian-fitted.
(27) Maximum value of the gradient of the OMWE.
(28) Minimum value of the gradient of the OMWE.

The correction unit 115 corrects the blood pressure measured by the measurement unit 114. The correction unit 115 inputs the OMW information and the OMWE information extracted by the measurement unit 114 to the correction model 116. The correction model 116 is a learning model that outputs, as a correction value, an error in blood pressure measured by the measurement unit 114 with respect to blood pressure actually measured by the auscultation method when the OMW information and the OMWE information are input. The correction model 116 is stored in, for example, a storage unit housed in the main body 110. The correction unit 115 corrects the blood pressure measured by the measurement unit 114 by using the correction value acquired from the correction model 116.

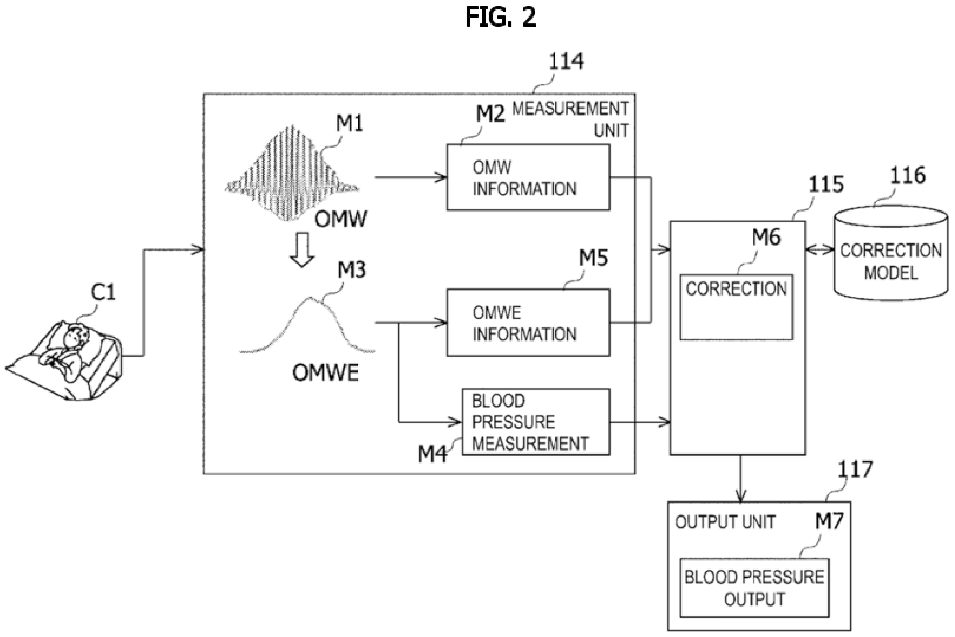

FIG. 2 is a diagram schematically illustrating correction of blood pressure by the correction unit 115 according to the embodiment. The correction of the blood pressure by the correction unit 115 will be described with reference to FIG. 2. The measurement unit 114 extracts the OMW of the measurement subject C1 (step M1). The measurement unit 114 extracts OMW information indicating the waveform of the OMW from the extracted OMW (step M2). The measurement unit 114 extracts the OMWE from the OMW (step M3), and measures the blood pressure of the measurement subject C1 with the oscillometric method based on the extracted OMWE (step M4). Further, the measurement unit 114 extracts OMWE information indicating a waveform of the OMWE from the OMWE (step M5).

The correction unit 115 inputs the OMW information extracted in step M2 and the OMWE information extracted in step M5 to the correction model 116, acquires an error of the blood pressure measured by the oscillometric method with respect to the blood pressure actually measured by the auscultation method, and corrects the blood pressure measured in step M4 based on the acquired error (step M6). The output unit 117 outputs the blood pressure corrected in step M6 to a display or the like (step M7).

Here, construction of the correction model 116 used for correction by the correction unit 115 will be briefly described. The correction model 116 is constructed by machine learning by using the OMW information and the OMWE information as the explanatory variables and by using the error of the blood pressure measured by the measurement unit 114 with respect to the blood pressure actually measured by the auscultation method as the objective variable.

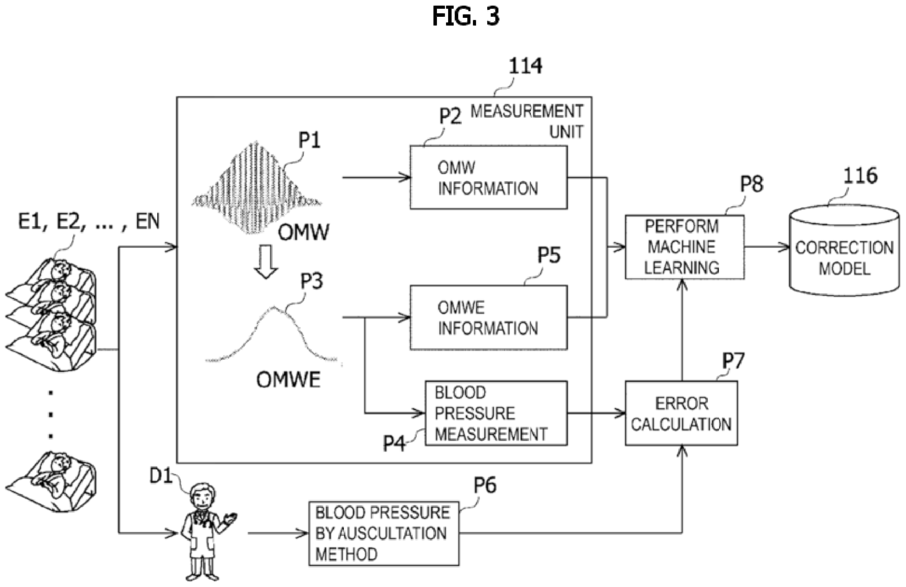

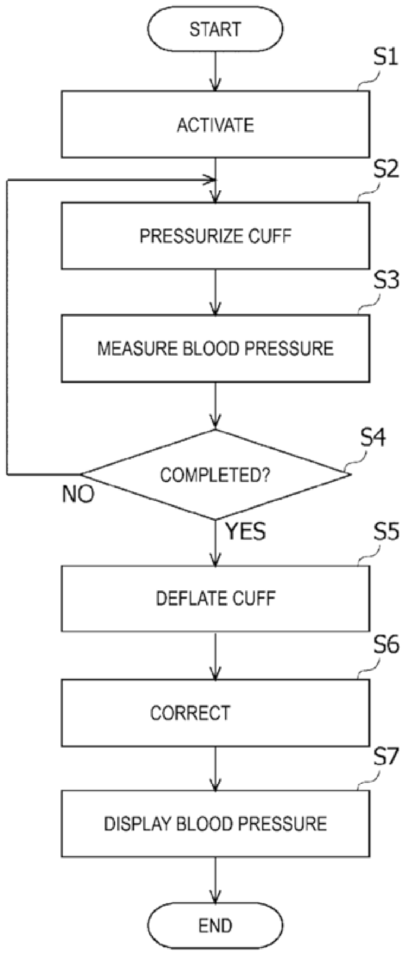

FIG. 3 is a diagram schematically illustrating the construction of the correction model 116 according to the embodiment. With reference to FIG. 3, the construction of the correction model 116 will be described. In the construction of the correction model 116, multiple subjects (E1, E2, ..., EN) are prepared. In the present embodiment, the multiple subjects are prepared, but the number of subjects may be one. The measurement unit 114 extracts the OMW of the subject E1 (step P1). The measurement unit 114 extracts OMW information indicating the waveform of the OMW from the extracted OMW (step P2). The measurement unit 114 extracts the OMWE from the OMW (step P3), and measures the blood pressure of the subject E1 based on the extracted OMWE with the oscillometric method (step P4). Further, the measurement unit 114 extracts OMWE information indicating a waveform of the OMWE from the OMWE (step P5).

A doctor D1 actually measures the blood pressure of the subject E1 by the auscultation method (step P6). The error of the blood pressure measured in step P4 with respect to the blood pressure actually measured in step P6 is calculated (step P7). The machine learning is performed with the OMW information extracted in step P2 and the OMWE information extracted in step P5 as explanatory variables and an error calculated in step P7 as the objective variable (step P8). The correction model 116 is constructed by the machine learning in step P8.

The processing from step P1 to P8 is also executed for each of the subjects E2 to EN. That is, the correction model 116 is not a learning model that increases the blood pressure measurement accuracy for one measurement subject, but is rather a model that increases the measurement accuracy of the blood pressure by the oscillometric method by using the measurement results of multiple subjects.

Returning to FIG. 1, the output unit 117 outputs the blood pressure corrected by the correction model 116. The output unit 117 is, for example, a Liquid Crystal Display (LCD), a Plasma Display Panel (PDP), an inorganic Electroluminescence (EL) panel, or an organic EL panel. The output unit 117 may be a printer or a speaker.

### <Processing Flow>

FIG. 4 is a diagram illustrating an example of a processing flow of the sphygmomanometer 100 according to the present embodiment. Hereinafter, an example of a processing flow of the sphygmomanometer 100 will be described with reference to FIG. 4.

In step S1, turning on the activation switch 111 activates the sphygmomanometer 100. In step S2, the control unit 112 inhales air into the cuff 120 to pressurize the arm of a measurement subject. The control unit 112 pressurizes with the cuff 120 to pressure higher than the maximum blood pressure, and then gradually lowers the pressure of the cuff 120.

In step S3, the measurement unit 114 extracts the OMW based on the cuff pressure detected by the pressure sensor 113, and extracts the OMW information from the extracted OMW. The measurement unit 114 extracts the OMWE from the OMW and extracts the OMWE information. The measurement unit 114 measures the blood pressure of the measurement subject by an oscillometric method using the OMWE.

In step S4, the measurement unit 114 determines whether or not the blood pressure measurement of the measurement subject has been completed. The measurement unit 114 may determine that the blood pressure measurement is completed, for example, when both the maximum blood pressure and the minimum blood pressure of the measurement subject have been measured. When completed (YES at step S4), the processing proceeds to step S5. When not completed (NO at step S4), the processing proceeds to step S2.

In step S5, the control unit 112 deflates the cuff 120. In step S6, the correction unit 115 inputs the OMW information and the OMWE information extracted in step S3 to the correction model 116, and acquires the error of the blood pressure measured by the oscillometric method with respect to the blood pressure actually measured by the auscultation method. The correction unit 115 corrects the blood pressure measured at step S3 by using the error acquired from the correction model 116.

In step S7, the output unit 117 outputs the blood pressure corrected in S6.

### <Verification>

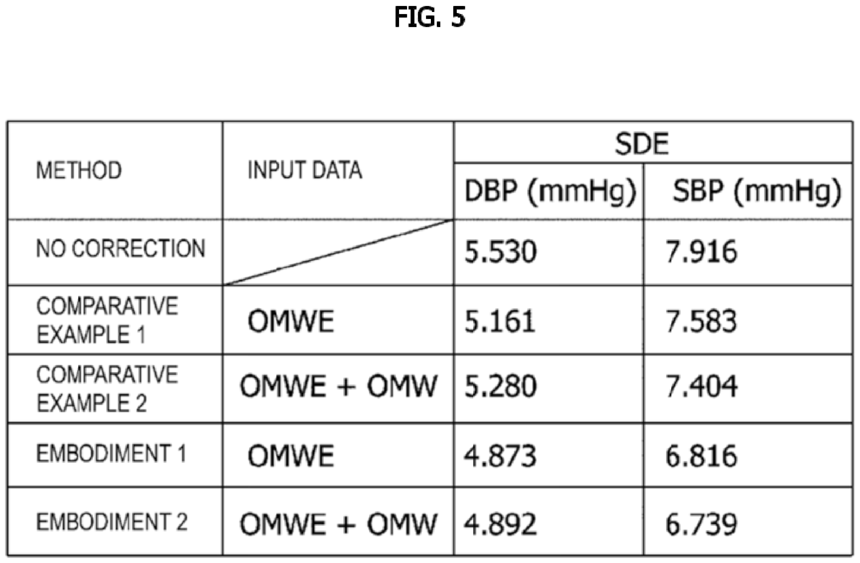

The accuracy of blood pressure measurement by the sphygmomanometer 100 according to the embodiment described above was verified, and will be described. FIG. 5 is a diagram exemplifying a verification result of the measurement accuracy of blood pressure. In FIG. 5, for the blood pressure (diastolic blood pressure (DBP), systolic blood pressure (SBP)) measured by the oscillometric method, in comparison with the embodiment, there are prepared a case which is not corrected with the correction model (no correction) and a comparative example in which the correction model is constructed by using the blood pressure as the objective variable. For each of the comparative example and the embodiment, there are prepared a case that the OMWE information is used as input data to the correction model (Comparative Example 1, Embodiment 1) and a case that both the OMWE information and the OME information are used (Comparative Example 2, Embodiment 2).

In the present verification, the measurement of the blood pressure was performed multiple times for multiple subjects, respectively, and the measurement results were acquired. In the present verification, the acquired measurement results are evaluated by Standard Deviation of Error (SDE).

Referring to FIG. 5, "Embodiment 1" and "Embodiment 2" showed higher accuracy than "No correction", "Comparative Example 1" and "Comparative Example 2". Referring to FIG. 5, even when the OMWE information is used or both the OMW information and the OMWE information are used as input data to the correction model, "Embodiment 1" and "Embodiment 2" showed higher accuracy than "No correction", "Comparative Example 1", and "Comparative Example 2".

### <Advantageous Effects of Embodiments>

According to the present embodiment, since the blood pressure measured by the measurement unit 114 is corrected by the correction unit 115, the measurement accuracy of the blood pressure can be increased as much as possible. Here, the correction model 116 uses the error of the blood pressure measured by the oscillometric method with respect to the blood pressure actually measured by the auscultation method as the objective variable. As described in the application example, the variable range of the error is about 1/10 of the variable range of the blood pressure. Since the number of pieces of training data tends to increase in proportion to the size of the variable range of the objective variable, according to the present embodiment in which the error is used as the objective variable, the number of pieces of training data used to construct the correction model 116 can be reduced as compared with the case that the blood pressure is used as the objective variable. That is, according to the present embodiment, it is possible to achieve blood pressure measurement with higher accuracy with less training data.

In the present embodiment, noise due to the body motion of the measurement subject, the way of winding the cuff, the position where the cuff is wound, and the like is also included in the OMW information and the OMWE information. Here, the correction model 116 is constructed by using the OMW information and the OMWE information as the explanatory variables. Therefore, the correction model 116 can output the error of the blood pressure measured by the oscillometric method with respect to the blood pressure actually measured by the auscultation method in consideration of the noise above. That is, according to the present embodiment, blood pressure measurement by the oscillometric method can be performed with higher accuracy.

Here, since pressure is applied to the human body in the blood pressure measurement by using the cuff, it is difficult to collect a large amount of data of the blood pressure measured by the oscillometric method, which is used in calculating the above error. In the present embodiment, the variable range of the objective variable can be narrowed by using the error as the objective variable, and thus the influence of the bias of the objective variable can be suppressed. Therefore, even when small amount of data of the blood pressure measured by the oscillometric method causes bias in the measured blood pressure data, the blood pressure measurement can be performed with higher accuracy.

### <First Modified Example>

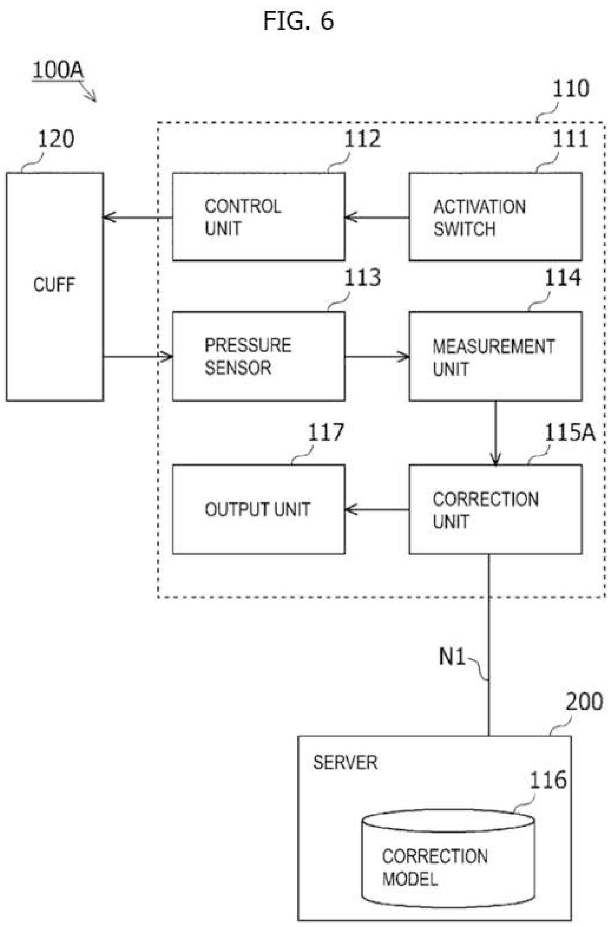

In the embodiment described above, the correction model 116 is stored in the storage unit in the main body 110. However, the correction model 116 may be stored in a portion other than the storage unit in the main body 110. FIG. 6 is a diagram illustrating an example of a sphygmomanometer 100A according to a first modified example. The sphygmomanometer 100A according to the first modified example is different from the sphygmomanometer 100 according to the embodiment in that the correction model 116 is stored not in the storage unit in the main body 110, but in a server 200 connected to the main body 110 via a computer network N1, and that a correction unit 115A is included instead of the correction unit 115.

The computer network N1 is a network that connects information processing devices to each other. The computer network N1 may be a wired network or a wireless network. The computer network N1 is, for example, the Internet.

The correction unit 115A accesses the correction model 116 stored in the server 200 via the computer network N1. That is, the correction unit 115A transmits the extracted OMW information and OMWE information to the server 200 via the computer network N1.

The server 200 inputs the OMW information and the OMWE information received from the sphygmomanometer 100A to the correction model 116, and acquires the error of the blood pressure measured by the oscillometric method with respect to the blood pressure measured by the auscultation method. The server 200 transmits the error to the main body 110 via the computer network N1. The correction unit 115A corrects the blood pressure measured by the measurement unit 114 by using the error received from the server 200.

According to the first modified example, the server 200 can execute the arithmetic processing of calculating the error by using the correction model 116. Therefore, the calculation load in the main body 110 can be reduced.

### <Other Modifications>

In the embodiment described above, the pressurization type blood pressure measurement has been described with reference to FIG. 4, but the sphygmomanometer 100 according to the present embodiment may perform depressurization type blood pressure measurement.

The embodiments and modified examples disclosed above can be combined.

### <Computer-Readable Recording Medium>

An information processing program for causing a computer or any other machine or device (hereinafter referred to as a computer or the like) to implement any of the functions described above may be recorded in a recording medium readable by the computer or the like. The functions can be provided by causing the computer or the like to read and execute the program from the recording medium.

Here, the term "computer-readable recording medium" refers to a recording medium that stores information such as data or programs by electrical, magnetic, optical, mechanical, or chemical means, and that can be read by a computer or the like. Examples of such recording medium that is removable from the computer or the like include a flexible disk, a magneto-optical disk, a Compact Disc Read Only Memory (CD-ROM), a Compact Disc-Recordable (CD-R), a Compact Disc-Rewritable (CD-RW), a Digital Versatile Disc (DVD), a Blu-ray disc (BD), a Digital Audio Tape (DAT), a 8mm tape, a flash memory, an external type hard disk drive, and a Solid State Drive (SSD). Examples of a recording medium fixed to the computer or the like include a built-in type hard disk drive, an SSD, and a ROM.

### <Supplementary Note 1>

A sphygmomanometer (100) including:
a cuff (120) to be wound around an arm of a measurement subject;
a control unit (112) configured to control pressure applied to the arm by the cuff (120);
a detection unit (113) configured to detect cuff pressure received by the cuff (120) from the arm;
a measurement unit (114) configured to measure blood pressure of the measurement subject by an oscillometric method by using a time-series change in the cuff pressure;
a correction model (116), for a subject, constructed by machine learning using information of a waveform related to the time-series change in the cuff pressure as an explanatory variable and using an error of blood pressure measured by the measurement unit with respect to blood pressure adopted as a true value as an objective variable;
a correction unit (115) configured to correct the blood pressure measured by the measurement unit (114) based on an error output by the correction model (116) by inputting the information of the waveform related to the time-series change in the cuff pressure detected by the detection unit (113) to the correction model (116), and
an output unit (117) configured to output the blood pressure corrected by the correction unit (115).

### <Supplementary Note 2>

The sphygmomanometer (100) according to Supplementary Note 1, in which
the blood pressure adopted as the true value is blood pressure actually measured by an auscultation method.

### <Supplementary Note 3>

The sphygmomanometer (100) according to Supplementary Note 1, in which
the information of the waveform includes information related to the cuff pressure.

### <Supplementary Note 4>

The sphygmomanometer (100) according to any one of Supplementary Notes 1 to 3, in which
the measurement unit (114) acquires an OscilloMetric Waveform (OMW) based on the time-series change in the cuff pressure, and
the information of the waveform includes OMW information indicating a waveform of the OMW.

### <Supplementary Note 5>

The sphygmomanometer (100) according to any one of Supplementary Notes 1 to 4, in which
the measurement unit (114) acquires an OscilloMetric Waveform Envelope (OMWE) based on the OMW, and
the information of the waveform includes OMWE information indicating a waveform of the OMWE.

### <Supplementary Note 6>

The sphygmomanometer (100) according to Supplementary Note 4, in which
the correction unit (115) corrects the blood pressure measured by the measurement unit (114) based on an error output by the correction model (116) by inputting the time-series change in the cuff pressure and the OMW information to the correction model (116).

### <Supplementary Note 7>

The sphygmomanometer (100) according to Supplementary Note 5, in which
the correction unit (115) corrects the blood pressure measured by the measurement unit (114) based on an error output by the correction model (116) by inputting the time-series change in the cuff pressure and the OMWE information to the correction model (116).

### <Supplementary Note 8>

A blood pressure measurement method comprising:
controlling, by a computer, pressure applied to an arm of a measurement subject by a cuff (120) wound around the arm;
detecting, by the computer, cuff pressure received by the cuff (120) from the arm;
   measuring, by the computer, blood pressure of the measurement subject by an oscillometric method by using a time-series change in the cuff pressure;
   correcting, by the computer for a subject, the blood pressure measured in the measuring based on an error output by a correction model (116), by inputting the information of the waveform related to the time-series change in the cuff pressure detected in the detecting to the correction model constructed by machine learning using information of a waveform related to the time-series change in the cuff pressure as an explanatory variable and using an error of blood pressure measured in the measuring with respect to blood pressure adopted as a true value as an objective variable; and
   outputting, by the computer, the blood pressure corrected in the correcting.

### <Supplementary Note 9>

A blood pressure measurement program for causing a computer to execute:
controlling pressure applied to an arm of a measurement subject by a cuff (120) wound around the arm,
detecting cuff pressure received by the cuff (120) from the arm;
measuring blood pressure of the measurement subject by an oscillometric method by using a time-series change in the cuff pressure;
correcting, for a subject, the blood pressure measured in the measuring based on an error output by a correction model (116), by inputting the information of the waveform related to the time-series change in the cuff pressure detected in the detecting to the correction model constructed by machine learning using information of a waveform related to the time-series change in the cuff pressure as an explanatory variable and using an error of the blood pressure measured in the measurement with respect to blood pressure adopted as a true value as an objective variable; and
outputting the blood pressure corrected in the correcting.

### <Supplementary Note 10>

A learning method generation method comprising:
receiving, by a computer, an input of an error of blood pressure measured by an oscillometric method with respect to blood pressure adopted as a true value; and
generating, by the computer, a learning model by machine learning by using information of a waveform related to a time-series change in cuff pressure by a cuff wound around an arm of a measurement subject as an explanatory variable and the error as an objective variable.

### <Supplementary Note 11>

A learning model generation program for causing a computer to execute:
receiving an input of an error of blood pressure measured by an oscillometric method with respect to blood pressure adopted as a true value; and
generating a learning model by machine learning by using information of a waveform related to a time-series change in cuff pressure by a cuff wound around an arm of a measurement subject as an explanatory variable and the error as an objective variable.

### REFERENCE SIGNS

100 Sphygmomanometer
100A Sphygmomanometer
110 Main body
111 Activation switch
112 Control unit
113 Pressure sensor
114 Measurement unit
115 Correction unit
115A Correction unit
116 Correction model
117 Output unit
120 Cuff
200 Server
C1 Measurement subject
D1 Doctor
E1 Subject
N1 Computer network

## Claims

1. A sphygmomanometer, comprising:
a cuff to be wound around an arm of a measurement subject;
a control unit configured to control pressure applied to the arm by the cuff;
a detection unit configured to detect cuff pressure received by the cuff from the arm;
a measurement unit configured to measure blood pressure of the measurement subject by an oscillometric method by using a time-series change in the cuff pressure;
a correction model, for a subject, constructed by machine learning using information of a waveform related to the time-series change in the cuff pressure as an explanatory variable and using an error of blood pressure measured by the measurement unit with respect to blood pressure adopted as a true value as an objective variable;
a correction unit configured to correct the blood pressure measured by the measurement unit based on an error output by the correction model by inputting the information of the waveform related to the time-series change in the cuff pressure detected by the detection unit to the correction model; and
an output unit configured to output the blood pressure corrected by the correction unit.

2. The sphygmomanometer according to claim 1, wherein
the blood pressure adopted as the true value is blood pressure actually measured by an auscultation method.

3. The sphygmomanometer according to claim 1 wherein,
the information of the waveform includes information related to the cuff pressure.

4. The sphygmomanometer according to any one of claims 1 to 3, wherein
the measurement unit acquires an OscilloMetric Waveform (OMW) based on a time-series change in the cuff pressure, and
the information of the waveform includes OMW information indicating a waveform of the OMW.

5. The sphygmomanometer according to claim 4, wherein
the measurement unit acquires an OscilloMetric Waveform Envelope (OMWE) based on the OMW, and
the information of the waveform includes OMWE information indicating a waveform of the OMWE.

6. The sphygmomanometer according to claim 4, wherein
the correction unit corrects the blood pressure measured by the measurement unit based on an error output by the correction model by inputting the time-series change in the cuff pressure and the OMW information to the correction model.

7. The sphygmomanometer according to claim 5, wherein
the correction unit corrects the blood pressure measured by the measurement unit based on an error output by the correction model by inputting the time-series change in the cuff pressure and the OMWE information to the correction model.

8. A blood pressure measurement method comprising:
controlling, by a computer, pressure applied to an arm of a measurement subject by a cuff wound around the arm;
detecting, by the computer, cuff pressure received by the cuff from the arm;
measuring, by the computer, blood pressure of the measurement subject by an oscillometric method by using a time-series change in the cuff pressure;
correcting, by the computer for a subject, the blood pressure measured in the measuring based on an error output by a correction model, by inputting the information of the waveform related to the time-series change in the cuff pressure detected in the detecting to the correction model constructed by machine learning using information of a waveform related to the time-series change in the cuff pressure as an explanatory variable and using an error of blood pressure measured in the measuring with respect to blood pressure adopted as a true value as an objective variable; and
outputting, by the computer, the blood pressure corrected in the correcting.

9. A blood pressure measurement program for causing a computer to execute:
controlling pressure applied to an arm of a measurement subject by a cuff wound around the arm;
detecting cuff pressure received by the cuff from the arm;
measuring blood pressure of the measurement subject by an oscillometric method by using a time-series change in the cuff pressure;
correcting, for a subject, the blood pressure measured in the measuring based on an error output by a correction model, by inputting the information of the waveform related to the time-series change in the cuff pressure detected in the detecting to the correction model constructed by machine learning using information of a waveform related to the time-series change in the cuff pressure as an explanatory variable and using an error of blood pressure measured in the measuring with respect to blood pressure adopted as a true value as an objective variable; and
outputting the blood pressure corrected in the correcting.

10. A learning model construction method comprising:
receiving, by a computer, an input of an error of blood pressure measured by an oscillometric method with respect to blood pressure adopted as a true value; and
constructing, by the computer, a learning model by machine learning by using information of a waveform related to a time-series change in cuff pressure by a cuff wound around an arm of a measurement subject as an explanatory variable and the error as an objective variable.

11. A learning model construction program for causing a computer to execute:
receiving an input of an error of blood pressure measured by an oscillometric method with respect to blood pressure adopted as a true value; and
constructing a learning model by machine learning by using information of a waveform related to a time-series change in cuff pressure by a cuff wound around an arm of a measurement subject as an explanatory variable and the error as an objective variable.
